# FASCICULE DE BREVET EUROPEEN

(11) **EP 3 720 391 B1**
(45) Date de publication et mention de la délivrance du brevet: **08.10.2025**
(21) Numéro de dépôt: 18830923.1
(22) Date de dépôt: 05.12.2018
(51) Int. Cl.: A61F 2/30, A61F 2/38, A61B 34/10

(54) **PROCEDE DE FABRICATION D'UN IMPLANT SUR-MESURE**
VERFAHREN ZUR HERSTELLUNG EINES MASSGESCHNEIDERTEN IMPLANTATS
METHOD FOR PRODUCING A TAILOR-MADE IMPLANT

(30) Priorité: 05.12.2017 FR 1761629
(43) Date de publication de la demande: 14.10.2020
(73) Titulaire: 3D Medical, 94440 Marolles-En-Brie (FR)
(72) Inventeur: GEMON, Jean-Pierre, 33000 Bordeaux (FR); NUTTENS, Vincent, 91800 Brunoy (FR)
(74) Mandataire: Plasseraud IP
(86) Numéro de dépôt international: PCT/FR2018/053117
(87) Numéro de publication internationale: WO 2019/110928

(56) Documents cités:
- WO-A1-2016/012730
- US-A1- 2011 144 760
- US-A1- 2016 256 279
- US-A1- 2017 172 747

## Description

### DOMAINE TECHNIQUE

La présente invention a pour objet un procédé de fabrication d'un implant sur-mesure, un programme d'ordinateur comportant des instructions pour l'exécution du procédé de fabrication dudit implant sur-mesure, un support d'enregistrement stockant ledit programme d'ordinateur, un support d'enregistrement sur lequel est stocké le programme d'ordinateur et une installation pour la fabrication d'un implant sur-mesure.

### ETAT DE LA TECHNIQUE

Une articulation est principalement composée :
- de cartilage, ni innervé, ni vascularisée, résistant mal à l'usure ;
- de synoviale, richement vascularisée, qui nourrit le cartilage et lubrifie l'articulation ;
- de ligaments, des tendons et des muscles permettant le soutien de l'articulation.

Les maladies ostéoarticulaires représentent environ 10% de l'ensemble des pathologies identifiées en France chaque année [Bulletin d'information en économie de la santé n°111(2006)]. Ces maladies inflammatoires et dégénératives des articulations sont pour la plupart consécutives au vieillissement ou à un traumatisme. Elles évoluent vers l'usure des cartilages ce qui se traduit par un handicap sévère. Pour l'instant aucun traitement permettant la réparation du tissu cartilagineux n'est vraiment disponible, hormis la pose d'un implant articulaire (arthroplastie) permettant de redonner la mobilité à l'articulation, l'arthrodèse permettant de rendre l'articulation immobile et l'ostéotomie afin de réorienter l'axe mécanique.

A titre d'exemple, une de ces pathologies est l'arthrose, maladie articulaire la plus répandue. L'arthrose est un problème de santé publique avec une prévalence en constante augmentation : 15% en 1990 à 18% prévue en 2020.

L'arthrose provoque d'importantes limitations fonctionnelles, une diminution de la qualité de vie et un important retentissement psychologique. Dans l'arthrose, on constate une détérioration, non seulement du cartilage, mais également de toutes les composantes articulaires (l'os, la synoviale, les tendons, les muscles).

Les genoux, les hanches, le rachis lombaire sont les premiers atteints par ce processus dégénératif car ce sont les régions articulaires les plus soumises au poids du corps et à des surcharges pondérales. Les doigts, le rachis cervical, l'épaule subissent également ce processus dégénératif, lié cette fois-ci à l'hyper mobilité répétitive de l'articulation. Cette pathologie peut toutefois toucher n'importe quelle articulation. On citera par ailleurs les processus dégénératifs de l'appareil locomoteur.

On citera par ailleurs d'autres pathologies telle que l'arthrite, la polyarthrite rhumatoïde, la lombalgie, l'ostéoporose, les troubles musculo-squelettiques.

Un des traitements de ces pathologies articulaires consiste à remplacer les surfaces articulaires lésées par des implants. Les implants utilisés dans ce cas nécessitent selon les techniques actuelles une résection importante des surfaces articulaires, cette résection se traduisant soit par la réalisation d'une surface plane à l'extrémité de l'os concerné, soit par la réalisation de plusieurs surfaces planes angulées les unes par rapport aux autres constituant une approximation de la surface réelle d'articulation.

Ce type d'élément de prothèse nécessite pour sa mise en place une résection importante de l'os. Cette résection nécessite à son tour la mise en œuvre d'un matériel chirurgical lourd pour définir précisément le plan ou les plans de la surface de résection à réaliser. En outre, la surface de résection étant plane ou constituée par plusieurs portions de plan, il est nécessaire de prévoir un ancrage très important en profondeur de l'élément de prothèse dans la partie de l'os associé à la surface articulaire pour assurer la solidarisation de la prothèse. Or les éléments d'ancrage tels que vis, broches, plots, etc... nécessitent le forage de trous d'ancrage importants dans l'os à traiter. Ce forage ou perçage important nécessite à son tour la mise en œuvre d'outils spécifiques. En outre, l'importance du forage ou du perçage dans l'os peut Il existe ainsi un besoin d'un procédé de fabrication d'un implant permettant d'obtenir un implant sur-mesure, respectant les structures anatomiques ainsi que les formes complexes des articulations de chaque individu et permettant ainsi d'adapter l'implant à la morphologie et/ou pathologie de l'individu.

### OBJET DE L'INVENTION

La présente invention vise à pallier les inconvénients de l'art antérieur et à répondre aux contraintes ci-dessus énoncées en proposant un procédé de fabrication d'un implant sur-mesure.

En effet, la présente invention concerne un procédé de fabrication d'un implant sur-mesure destiné à être implanté sur un site de pose d'une partie osseuse lésée, le procédé comprenant une étape de superposition par fondu d'une représentation 3D d'un implant standard sur une représentation 3D d'une partie osseuse lésée en positionnant ledit implant standard sur un site de pose de la partie osseuse lésée, afin d'éventuellement procéder à des modifications des dimensions et/ou ajustement de la forme dudit implant standard, et éventuellement également modifier la surface externe entrainer des conséquences dommageables pour la partie de l'os qui a subi cette perforation, notamment en ce qui concerne sa résistance mécanique lorsque des contraintes sont appliquées aux os constituant cette articulation lors de mouvements effectués puisque, par sa longueur, l'élément d'ancrage reporte l'effort en une zone de l'os qui n'est pas prévue pour supporter cet effort.

De plus, dans le cas d'une résection importante sensiblement plane, l'appui de la prothèse sur la surface de résection est non satisfaisant car cette surface est essentiellement constituée par de l'os spongieux, l'appui sur la périphérie corticale de l'os étant insuffisant quant aux reports d'effort.

Enfin, les implants connus n'offrent pas aux praticiens un choix suffisant de gamme pour répondre aux degrés de liberté articulaire choisis, lesdits implants étant standardisés.

Le document US2011/144760, selon son abstract, décrit des méthodes et des dispositifs de réparation d'une articulation du genou, qui comprennent des implants personnalisables et/ou hautement sélectionnables et/ou des composants d'implant pour chaque patient destiné à procurer un fonctionnement et un ajustement optimaux.

Le document WO2016/012730, selon son abstract, décrit un procédé de conception assistée par ordinateur d'un implant sur mesure pour une articulation de deux os d'un patient, qui comprend des étapes consistant à: positionner un volume virtuel d'encombrement prothétique dans l'espace du modèle virtuel, et par rapport à un repère anatomique de référence de ladite articulation, de manière à définir deux zones d'intersection entre ledit volume virtuel d'encombrement prothétique et l'articulation des deux os, lesdites deux zones d'intersection définissant des plans de coupes osseuses nécessaires à la mise en place de l'implant; détecter les contours desdites zones d'intersection; concevoir l'implant sur mesure à partir d'un implant standard, auquel on adapte les contours des surfaces d'appui dudit implant standard avec les contours préalablement détectés qui correspondent à des préparations osseuses pour une implantation de l'implant. dudit implant standard, pouvant être, soit l'empreinte ou sensiblement l'empreinte de la surface externe de ladite partie osseuse en état avant superposition dudit implant lorsque la géométrie de la partie osseuse lésée est destinée à être conservée, soit une surface externe fonctionnelle, lorsque ledit implant sur-mesure est destiné à être mis en œuvre à l'interface de deux parties osseuses coopérant l'une avec l'autre.

La présente invention a également pour objet un procédé de fabrication d'un implant sur-mesure destiné à être implanté sur un site de pose d'une partie osseuse lésée, le procédé comprenant une étape selon laquelle une modification locale a été directement réalisée sur un site de pose de la représentation graphique en 3D de ladite partie osseuse lésée, la forme et les dimensions de la surface d'ancrage de l'implant étant fonction de la forme et des dimensions de ladite au moins une modification locale afin de permettre l'ancrage dudit implant sur ladite partie osseuse à traiter, la surface externe étant par la suite déterminée.

Ainsi, et avantageusement, la surface d'ancrage dudit implant sur-mesure obtenu par le procédé selon l'invention n'est pas plane et épouse le site de pose à la surface de ladite partie osseuse lésée, soit en modifiant les dimensions et/ou ajustant la forme d'un implant standard en tenant compte d'au moins un paramètre de la partie osseuse lésée, soit en déterminant les formes et les dimensions de ladite surface d'ancrage par rapport aux formes et dimensions de ladite au moins une modification locale réalisée sur la représentation graphique de la partie osseuse lésée.

La surface osseuse lésée est donc avantageusement préservée. Le procédé selon l'invention permet d'éviter une résection importante et non nécessaire de l'os et de ne déterminer que les résections nécessaires à la différence des techniques antérieures, typiquement de la coupe par sciage, selon laquelle l'ancrage de l'implant se fait plan sur plan.

Nous notons qu'il ressort clairement de l'objet de l'invention que la détermination de l'implant sur mesure est obtenue à partir de la ou des seules images acquises lors d'une étape liminaire (étape i) et qu'aucune action n'est réalisée à aucun moment que ce soit sur le corps du patient.

Ainsi la surface d'ancrage de l'implant sur-mesure fabriqué par le procédé selon l'invention prend la place exacte des structures osseuse évidées, gage d'une répartition saine des contraintes et d'une immobilisation primaire de l'implant sans micro-mouvements et garantissant ainsi une fixation durable et une reconstruction osseuse.

Avantageusement encore, le procédé selon l'invention permet de déterminer la surface externe de l'implant sur-mesure, cette surface externe étant soit l'empreinte ou sensiblement l'empreinte de la surface externe de ladite partie osseuse lorsque la géométrie de la partie osseuse lésée est destinée à être conservée, soit une surface fonctionnelle, lorsque ledit implant sur-mesure est destiné à être mis en œuvre à l'interface de deux parties osseuses coopérant l'une avec l'autre.

Ainsi, et dans un cas, le procédé de fabrication de l'implant sur-mesure permet la reconstruction de la surface externe de la partie osseuse lésée, et dans un autre cas, la surface externe est une surface fonctionnelle déterminée en fonction de la surface conjuguée d'une autre partie osseuse avec laquelle ladite partie osseuse à réparer coopère de manière à soit redonner la mobilité à l'articulation dans le cadre d'une arthroplastie, soit rendre l'articulation immobile dans le cadre d'une arthrodèse, soit réorienter l'axe mécanique dans le cadre d'une ostéotomie, afin d'obtenir un implant sur-mesure permettant la correction anatomique d'une pathologie et adapté aux propriétés physiques et mécaniques des surfaces osseuses.

Le procédé selon l'invention offre donc un choix d'implants sur-mesure répondant aux degrés de liberté articulaire et adapté à chaque morphologie et pathologie.

Le procédé selon l'invention permet en outre une économie de temps au bloc opératoire, une économie de coût ainsi qu'une économie de capital osseux.

### DESCRIPTION DETAILLEE DE L'INVENTION

Ainsi, l'invention concerne un procédé de fabrication d'un implant sur-mesure tel que défini dans la revendication 1.

On entend par « site de pose » au sens de la présente invention, au moins une zone de la partie osseuse qui va être retirée de manière à recevoir l'implant.

Le site de pose peut être local ou sur toute la surface de la partie osseuse. Le site de pose peut être uni, bi ou tri compartimental. Ainsi, le site de pose correspond aux configurations possibles de la reprise de la surface osseuse.

Typiquement, la reprise osseuse peut aller de 10 à 100% de reprise osseuse.

Typiquement, la reprise osseuse peut être totale (100% de reprise osseuse), et la totalité de la partie osseuse est récupérée.

Ainsi, l'implant sur-mesure peut être un implant uni, bi ou tricompartimental.

A titre purement illustratif, un implant pourra être considéré comme unicompartimental dans le cadre d'une reprise osseuse jusqu'à environ 30% de la partie osseuse, comme bicompartimental dans le cadre d'une reprise osseuse comprise entre environ 30 % et 60% de la partie osseuse et comme tricompartimental dans le cadre d'une reprise osseuse comprise entre environ 60% et 100% de la partie osseuse.

On entend par « partie osseuse » au sens de la présente invention, l'extrémité d'un os ou la surface externe d'une partie d'un os.

Typiquement, les parties osseuses pourront être choisies parmi les os composant le genou tels que le tibia et le fémur, les vertèbres lombaires et cervicales, l'acromion de l'omoplate, la tête de l'humérus, la clavicule, les os composant le pied, les os composant la cheville, les os composant le bassin, les os composant la hanche, le rachis lombaire, le rachis cervical, les os composant l'épaule, les os composant le coude, les os composant le poignet, les os composant la main, les dents.

Dans un mode préféré de l'invention, les parties osseuses coopèrent l'une avec l'autre et forment une articulation.

A titre purement illustratif, on citera, les articulations intervertébrales, l'articulation lombo sacrée, l'articulation sacro-coccygienne, les articulations inter coccygiennes, les articulations sacro-iliaques, la symphyse pubienne, l'articulation gléno-humérale, l'articulation acromio-claviculaire, l'articulation huméro-ulnaire, l'articulation huméro-radiale, l'articulation radio-ulnaire proximale, l'articulation radio-carpienne, l'articulation radio-ulnaire distale, les articulations entre les os du carpe, les articulations carpo-métacarpiennes, les articulations inter métacarpiennes, les articulations métacarpo-phalangiennes, les articulations inter phalangiennes, l'articulation coxo-fémorale, l'articulation tibio-fémorale, l'articulation patello-fémorale, l'articulation tibiofibulaire proximale, l'articulation talo-crurale, l'articulation tibiofibulaire distale, les articulations entres les os du tarse, les articulations tarsométatarsiennes, les articulations inter-métatarsiennes, les articulations métatarso-phalangiennes, les articulations interphalangiennes.

Préférentiellement, les deux parties coopérant l'une avec l'autre seront le tibia et le fémur, préférentiellement l'extrémité distale du fémur et l'extrémité proximale du tibia.

On entend par « partie osseuse lésée » toute partie osseuse ayant subi un traumatisme, une dégradation, une dégénérescence, une inflammation, une désorganisation du tissu osseux, un processus dégénératif.

Typiquement, les paramètres de la partie osseuse lésée pourront être choisis parmi les propriétés densitométriques tels que la porosité, la densité, les propriétés mécaniques telles que ses propriétés élastiques, ses propriétés viscoélastiques.

On entend par « surface externe » de l'implant, la partie de l'implant qui n'est pas destinée à être en contact directement avec la surface osseuse recevant l'implant.

On entend par « surface externe fonctionnelle », la partie de l'implant qui n'est pas destinée à être en contact avec la surface osseuse recevant l'implant et qui est destinée à être mis en œuvre à l'interface de deux parties osseuses coopérant l'une avec l'autre, de manière, par exemple à créer le couple de frottement.

La surface externe fonctionnelle est une surface fonctionnelle qui représente une surface de l'implant qui vient coopérer avec une autre surface fonctionnelle d'une autre partie osseuse pour créer le couple de frottement. La surface fonctionnelle représente donc une surface de frottement qui donne la fonctionnalité à l'articulation constituée par une partie osseuse saine et une partie osseuse lésée.

De manière générale, les articulations comprennent une ou plusieurs surfaces fonctionnelles qui donnent la mobilité et la morphologie propres à l'individu. La forme et le nombre de ces surfaces varient donc en fonction de la capacité de l'articulation de pouvoir répondre à différents degrés de mobilité fonctionnelle.

Dans l'art antérieur, les implants obtenus ne permettent pas répondre à la spécificité des surfaces fonctionnelles de l'implant en fonction de l'articulation, et donc à la spécificité de la mobilité et de la morphologie de chaque individu. De ce fait, à titre d'exemple, dans le cas d'une articulation de genou, les implants standards entraînent certaines conséquences gênantes pour le patient : luxation tibiale, coupe de la pente tibiale et décalage et changement de la mobilité et de la stabilité de l'articulation.

Le procédé de la présente invention permet de surmonter ces inconvénients en proposant un implant avec des surfaces fonctionnelles qui correspondent à celles de la partie osseuse lésée de manière à préserver ou retrouver la mobilité et la morphologie de l'individu avant la mise en place de l'implant.

Le procédé de la présente invention propose d'extraire non seulement la surface d'ancrage mais également la surface fonctionnelle de l'implant sur-mesure à partir de la superposition par fondu d'une représentation 3D d'un implant standard sur la représentation 3D obtenue de la partie osseuse lésée. Plus précisément cette extraction a été réalisée lorsque la partie osseuse lésée était en position fonctionnelle avec l'autre partie osseuse.

Dans le cas où l'état de la partie osseuse lésée ne permet pas d'extraire la ou les surfaces fonctionnelles de l'implant, le procédé comprend une étape dans laquelle une image négative 3D est réalisée à partir de l'autre partie osseuse conjuguée de la partie osseuse lésée pour extraire la ou les surfaces fonctionnelles en tenant compte de la mobilité de l'individu.

Ainsi le procédé de la présente invention, en déterminant les surfaces fonctionnelles de l'implant, permet de reproduire le couple de frottement entre deux parties osseuses de l'articulation et permet :
- la conservation de l'intégralité de la morphologie ;
- la conservation du couple de frottement, et donc la mobilité
- la conservation intra et extra-articulaire des ligaments et des ménisques, cela permet de conserver la mobilité et la stabilité de l'individu.

On entend par « surface d'ancrage » au sens de la présente invention, la surface de l'implant destinée à être directement en contact avec la partie osseuse (site de pose) recevant l'implant.

On entend, au sens de la présente invention le terme « surface conjuguée » une surface pouvant par exemple correspondre, et à titre non limitatif, à la surface externe d'un autre implant reçu à la surface d'une deuxième partie osseuse ou correspondant à la surface d'une deuxième partie osseuse coopérant avec la première partie osseuse recevant l'implant.

### BREVE DESCRIPTION DES DESSINS

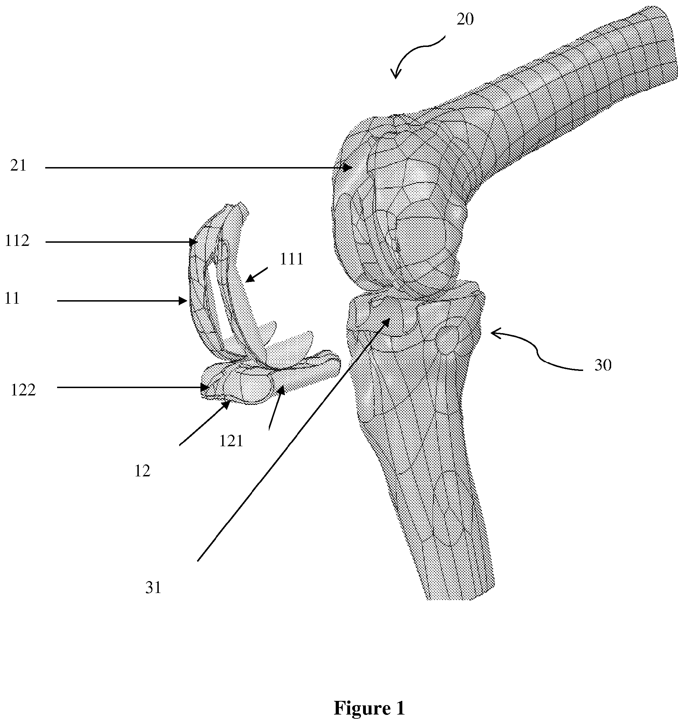

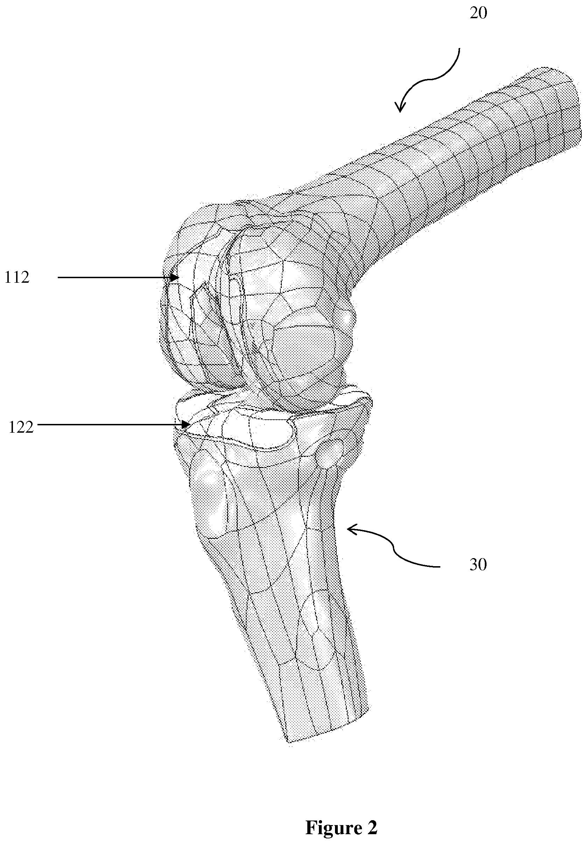

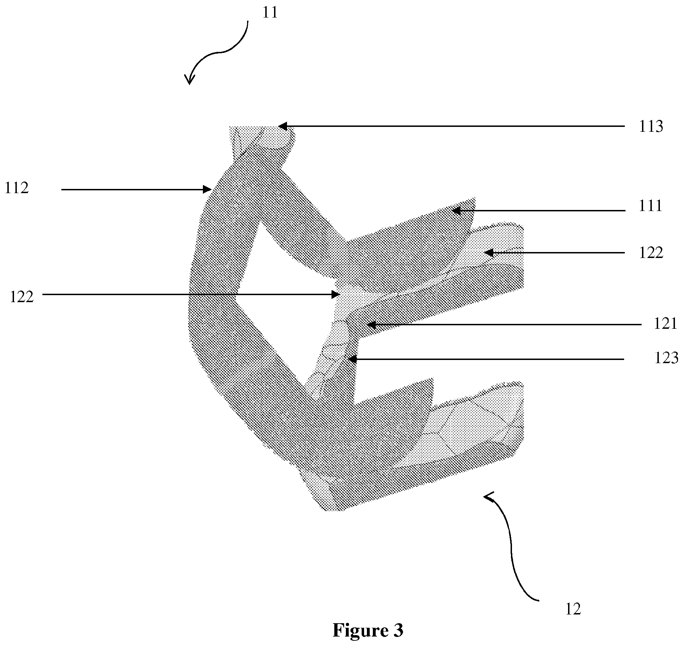

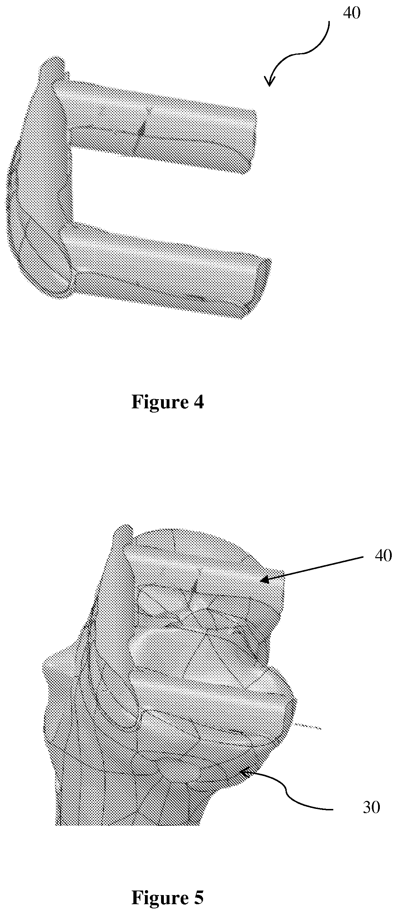

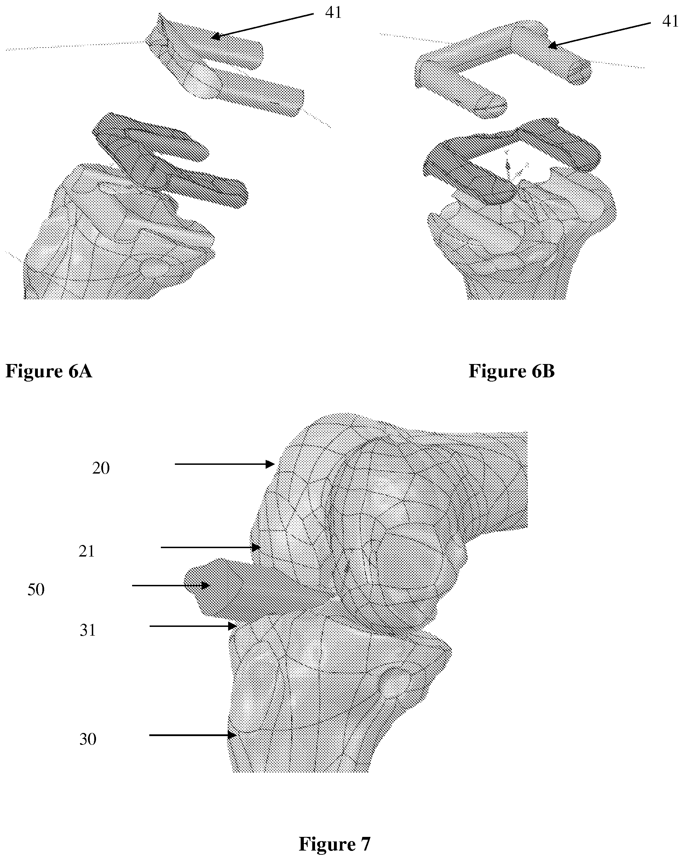

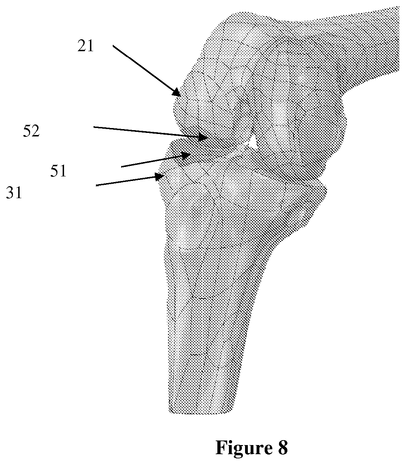

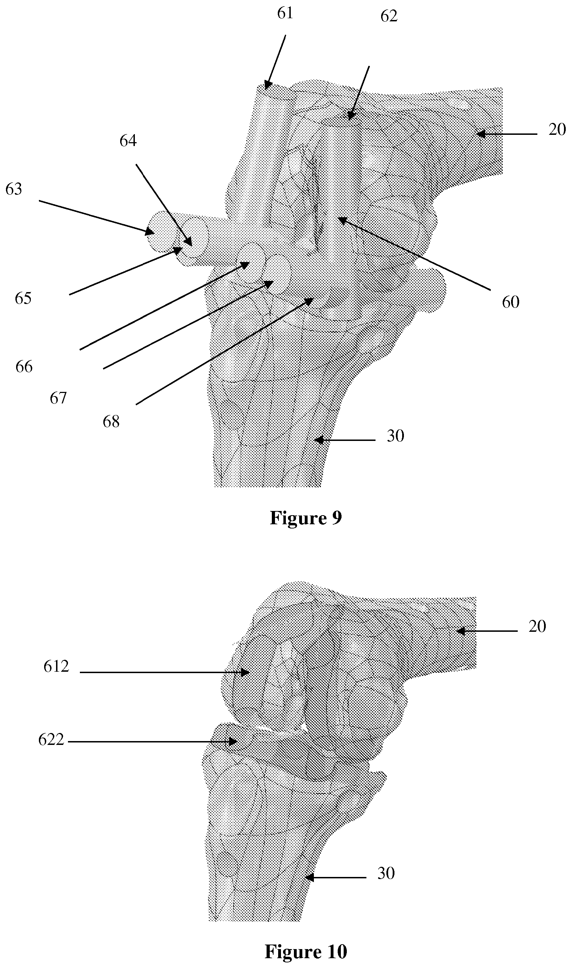

D'autres avantages, buts et caractéristiques particulières de la présente invention ressortiront de la description qui va suivre, faite, dans un but explicatif et nullement limitatif, en regard des dessins annexés, dans lesquels :
**LA** **FIGURE 1** est une vue en en perspective de deux implants sur-mesure fabriqués selon le procédé selon l'invention et de l'articulation tibio-fémorale.
**LA** **FIGURE 2** est une vue en perspective de deux implants sur-mesure fabriqués selon le procédé selon l'invention, lesdit implants étant ancrés dans l'articulation tibio-fémorale.
**LA** **FIGURE 3** est une vue en perspective de deux implants sur-mesure fabriqués selon le procédé selon l'invention.
**LA** **FIGURE 4** est une vue en perspective d'un implant standard choisi dans une bibliothèque. L'implant est un implant bicompartimental à 60%
**LA** **FIGURE 5** est une vue en perspective d'une représentation 3D d'une partie osseuse et de la superposition d'une représentation 3D d'un implant standard
**LES** **FIGURES 6A** **ET 6B** sont des vues en perspective d'une représentation 3D d'une partie osseuse en 3D et d'une représentation 3D d'un implant standard et de la modification des dimensions et/ou ajustement de la forme de la représentation 3D dudit implant standard ainsi que de la modification de la surface externe
**LA** **FIGURE 7** est une vue en en perspective d'une représentation 3D de l'articulation tibio-fémorale et d'une représentation 3D d'un implant standard dessiné spécifiquement pour les parties osseuses lésées
**LA** **FIGURE 8** est une vue en en perspective d'une représentation 3D de l'articulation tibio-fémorale et d'une représentation 3D d'un implant dessiné spécifiquement pour les parties osseuses lésées, les surfaces fonctionnelles de l'implant ayant été déterminées
**LA** **FIGURE 9** est une d'une représentation 3D de l'articulation tibio-fémorale et d'une représentation 3D d'un implant possédant des formes géométriques correspondant sensiblement aux modifications locales
**LA** **FIGURE 10** Est une représentation 3D de l'articulation tibio-fémorale et du site de pose une fois les formes et dimensions ainsi que les surfaces fonctionnelles de la représentation 3D de l'implant déterminées

### DESCRIPTION DETAILLEE DE L'INVENTION

Tout d'abord, on note que les figures ne sont pas à l'échelle.

Les images des parties osseuses, ici les parties osseuses coopérant dans l'articulation tibio-fémorale, ont été acquises par Imagerie par Résonnance Magnétique et ont été par la suite segmentées et modélisées en 3D sur un ordinateur, la transmission desdites images étant réalisée sous format STL. Une imagerie 3D de l'articulation tibio-fémorale, i.e. de l'extrémité proximale tibiale 30 et de l'extrémité distale fémorale 20 est ainsi acquise, et telle que par exemple représentée à la Figure 1, en position fonctionnelle.

Sur la base de cette imagerie 3D de l'articulation tibio-fémorale en position fonctionnelle, les évidements nécessaires pour accueillir les implants sur-mesure 11, 12 ont été réalisés sur l'imagerie 3D de l'articulation tibio-fémorale pour modifier localement les extrémités osseuses proximale tibiale 30 et distale fémorale 20.

Un évidement 21 a été réalisé sur l'extrémité distale fémorale 20 et un évidement 31 sur l'extrémité proximale tibiale 30.

Grace à chaque évidement 21, 31 la forme et les dimensions de chaque implant, respectivement 11 et 12, vont être déterminées de manière à ce que la surface d'ancrage 111, 121 de chaque implant soit l'empreinte de la zone retirée 21, 31 de l'extrémité distale fémorale 20 et de l'extrémité proximale tibiale 30.

Les implants sur-mesure 11 et 12 sont destinés à être mis en œuvre à l'interface de deux parties osseuses coopérant l'une avec l'autre, les surfaces externes 112 et 122 des implants 11 et 12 sont des surfaces fonctionnelles.

Ainsi, la surface fonctionnelle 112 de l'implant 11 va être déterminée en fonction de la surface conjuguée de l'extrémité proximale tibiale 30 avec laquelle l'extrémité distale fémorale 20 coopère et ce afin d'assurer l'articulation entre l'extrémité proximale tibiale 30 et l'extrémité distale fémorale 20, la surface conjuguée correspondant à la surface externe fonctionnelle 122 de l'implant 12.

De la même façon, la surface fonctionnelle 122 de l'implant 12 va être déterminée en fonction de la surface conjuguée de l'extrémité distale tibiale 20 avec laquelle l'extrémité proximale tibiale 30 coopère et ce afin d'assurer l'articulation entre l'extrémité proximale tibiale 30 et l'extrémité distale fémorale 20, la surface conjuguée correspondant à la surface externe fonctionnelle 112 de l'implant 11.

L'articulation entre l'extrémité proximale tibiale 30 et l'extrémité distale fémorale 20 est avantageusement récupérée.

Avantageusement le procédé de fabrication d'un implant sur-mesure selon la présente invention permettra de ne faire que la résection nécessaire de l'os et permet de préserver le capital osseux du patient et de recréer l'articulation tibio-fémorale (Figure 2).

Dans le présent mode de réalisation, l'extrémité distale fémorale 20 et proximale tibiale 30 ont été réalisé par fabrication additive afin de vérifier l'adéquation des implants.

La Figure 3 représente un dispositif médical sur-mesure composée d'un implant 11 destiné à être reçu sur l'extrémité distale 20 du fémur (non représentée) et un implant 12 destiné à être reçu sur l'extrémité proximale 30 du tibia (non représentée), les deux implants représentent une prothèse tricompartimentale obtenu par le procédé selon l'invention.

Chaque implant comprend une surface d'ancrage 111, 121 étant l'empreinte ou sensiblement l'empreinte d'au moins une modification locale sur une partie osseuse lésée, une surface fonctionnelle 112, 122 destiné à être mis en œuvre à l'interface des deux parties osseuses coopérant l'une avec l'autre afin d'assurer l'articulation du tibia et du fémur, et une protubérance 113, 123 reliant les deux pattes de chaque corps. La relation des surfaces externes 112 et 122 est le couple de frottement.

La surface d'ancrage de chaque implant comprend un relief de surface destiné à renforcer l'ancrage dudit implant. Ce relief interne est constitué de trabéculaires, qui sont des structures poreuses reprenant le design de l'os spongieux et permettant une reconstruction osseuse particulièrement efficace.

Les implants 11 et 12 sont réalisés en titane. Les trabéculaires des surfaces d'ancrage 111 et 121 des implants sont réalisés par projection plasma de suspension d'oxyde de titane.

Avantageusement, les implants selon l'invention et plus particulièrement la prothèse totale de genou selon ce mode particulier de l'invention sont des implants sur-mesure, à l'identique de la morphologie du patient et intégrant complètement le fonctionnement personnel du patient (déplacement / glissement / rotation), obtenus à partir du procédé selon l'invention.

La Figure 4 correspond à implant standard 40 choisi dans une bibliothèque. L'implant est un implant bicompartimental à 60%.

L'implant standard est modélisé en CAO.

La Figure 5 est une vue en perspective d'une représentation 3D d'une partie osseuse, ici l'extrémité proximale tibiale 30 et de la superposition d'une représentation 3D d'un implant standard 40.

Selon le procédé de fabrication d'un implant sur-mesure selon l'invention, et destiné à être implanté sur un site de pose d'une partie osseuse lésée, les images de l'extrémité proximale du tibia 30 ont été acquises par Imagerie par Résonnance Magnétique et ont été par la suite segmentées et modélisées en 3D sur un ordinateur, la transmission desdites images étant réalisée sous format STL. Une imagerie 3D de l'extrémité proximale tibiale 30 est ainsi acquise.

La représentation 3D d'un implant standard 40 a été superposée sur la représentation 3D de l'extrémité proximale du tibia 30, en positionnant ledit implant standard 40 sur un site de pose à la surface de l'extrémité proximale du tibia 30 lésée.

Selon la Figure 6, les dimensions de l'implant standard 40 vont être modifiées et la forme dudit implant standard 40 va être ajustée par soustraction de matière de l'implant.

La surface externe 41 dudit implant standard va être modifiée, de sorte à être l'empreinte ou sensiblement l'empreinte de la surface externe de ladite partie osseuse, occupée par ledit implant standard, en l'état avant superposition dudit implant, de manière à conserver la géométrie de la partie osseuse.

Ainsi et avantageusement, la géométrie de l'extrémité proximale va pouvoir être recréée.

La Figure 7 est une vue en en perspective d'une représentation 3D de l'articulation tibio-fémorale et d'une représentation 3D d'un implant standard dessiné spécifiquement pour les parties osseuses lésées.

De la même façon que précédemment, les images de l'extrémité proximale du tibia 30 et de l'extrémité distale fémorale 20 ont été acquises par Imagerie par Résonnance Magnétique et ont été par la suite segmentées et modélisées en 3D sur un ordinateur, la transmission desdites images étant réalisée sous format STL. Une imagerie 3D de l'extrémité proximale tibiale 30 et de l'extrémité distale fémorale 20 est ainsi acquise.

La représentation 3D d'un implant 50 dessinée selon une forme géométrique spécifiquement destinée aux parties osseuses lésées, c'est-à-dire le condyle latéral de l'extrémité distale du fémur 21 et le plateau latéral de l'extrémité proximale du tibia 31 a été superposée sur la représentation 3D sur un site de pose à la surface de chaque partie osseuse lésée, c'est-à-dire à la surface du condyle latéral de l'extrémité distale du fémur 21 et à la surface du plateau latéral de l'extrémité proximale du tibia.

Dans ce cas, la représentation 3D de l'implant ne correspond pas à un implant standard, mais à une représentation 3D d'un implant nouvellement crée déterminée par le praticien comme étant plus adapté d'un point de vue géométrique aux parties osseuses lésées.

Les formes et dimensions de la représentation 3D de l'implant vont être modifiées afin de remplacer sur la représentation 3D, la partie osseuse lésée à la surface du condyle latéral de l'extrémité distale du fémur et du plateau latéral de l'extrémité proximal du tibia par la représentation 3D de l'implant pour déterminer les limitations de l'implant ainsi que les limitations de l'ancrage de l'implant dans le condyle latéral fémoral et dans le plateau latéral tibial de manière à ce que l'implant soit la représentation conforme et personnalisé des parties osseuses soustraites et lésées.

L'implant est destiné à être mis en œuvre à l'interface de deux parties osseuses coopérant l'une avec l'autre, le condyle latéral de l'extrémité distale du fémur 21 et le plateau latéral de l'extrémité proximale du tibia 31.

Aussi, et une fois que la représentation 3D de l'implant 50 a été positionnée sur les sites de pose des parties osseuses lésées et les modifications des dimensions et l'ajustement de la forme de l'implant effectués pour correspondre à ces parties osseuses lésées à traiter, la surface externe de l'implant ancré dans le condyle latéral de l'extrémité distale du fémur 21 et la surface externe de l'implant ancré sur le plateau latéral de l'extrémité proximale tibiale vont être déterminées, les deux surfaces étant des surfaces fonctionnelles.

Ainsi, et tel que représenté à la Figure 8, la surface fonctionnelle 51 de la représentation 3D de l'implant ancré sur le plateau latéral 31 va être déterminée en fonction de la surface conjuguée du condyle latéral 21 avec lequel il coopère et ce afin d'assurer l'articulation entre le plateau latéral 31 et le condyle latérale 21, la surface conjuguée correspondant à la surface externe fonctionnelle de l'implant 52.

De la même façon, la surface fonctionnelle 52 de la représentation 3D de l'implant ancré sur le condyle latéral 21 va être déterminée en fonction de la surface conjuguée du plateau tibial 31 avec lequel il coopère et ce afin d'assurer l'articulation entre le plateau latéral 31 et le condyle latérale 21, la surface conjuguée correspondant à la surface externe fonctionnelle de l'implant 51.

L'articulation entre condyle latéral de l'extrémité distale du fémur 21 et le plateau latéral de l'extrémité proximale du tibia 31 est avantageusement récupérée.

**LA** **FIGURE 9** est la représentation 3D de l'articulation tibio-fémorale et d'une représentation 3D d'un implant possédant des formes géométriques correspondant sensiblement aux modifications locales.

De la même façon que précédemment, les images de l'extrémité proximale du tibia 30 et de l'extrémité distale fémorale 20 ont été acquises par Imagerie par Résonnance Magnétique et ont été par la suite segmentées et modélisées en 3D sur un ordinateur, la transmission desdites images étant réalisée sous format STL. Une imagerie 3D de l'extrémité proximale tibiale 30 et de l'extrémité distale fémorale 20 est ainsi acquise.

La représentation 3D d'un ancillaire 60 comprenant des axes de coupe déterminés chacun par l'axe longitudinal d'un tube de guidage d'un outil tel qu'un foret déterminant des zones de travail a été superposée sur la représentation 3D sur un site de pose à la surface de chaque partie osseuse lésée. Les axes de coupe de la représentation 3D de cet ancillaire correspondent aux modifications locales à apporter à la surface de chaque partie osseuse lésée.

Ainsi, la représentation 3D de l'ancillaire 60 possède huit élément tubulaire 61, 62, 63, 64, 65, 66, 67, 68, possédant chacun un axe longitudinal, chaque axe longitudinal de chacun des éléments tubulaires coïncidant avec un seul des huit axes de travail déterminés par le praticien sur la représentation 3D de l'articulation tibio-fémorale, ces axes de travail correspondant aux modifications locales à apporter aux parties osseuses lésées.

Par exemple, l'axe longitudinal de l'élément tubulaire 61 de la représentation 3D de l'ancillaire correspondra sensiblement à l'évidement passant par la partie inférieure du condyle latéral de l'extrémité distale fémorale.

L'axe longitudinal de l'élément tubulaire 62 de de la représentation 3D de l'ancillaire correspondra sensiblement à l'évidement passant par la partie inférieure du condyle médial de l'extrémité distale fémorale.

L'axe longitudinal de l'élément tubulaire 63 de la représentation 3D de l'ancillaire correspondra sensiblement à l'évidement dirigé vers la joue latérale de la trochlée.

L'axe longitudinal de l'élément tubulaire 64 de la représentation 3D de l'ancillaire correspondra sensiblement à l'évidement dirigé vers la zone de juxtaposition entre la partie postérieure du condyle latéral et de la surface glénoïde latérale du plateau tibial.

L'axe longitudinal de l'élément tubulaire 66 de la représentation 3D de l'ancillaire correspondra sensiblement à l'évidement dirigé vers la zone de juxtaposition entre la partie postérieure du condyle média et de la surface glénoïde média du plateau tibial.

L'axe longitudinal de l'élément tubulaire 65 de la représentation 3D de l'ancillaire correspondra sensiblement à l'évidemment dirigé vers la gorge (ou sillon) médiane de la trochlée.

L'axe longitudinal de l'élément tubulaire 67 de la représentation 3D de l'ancillaire correspondra sensiblement à l'évidement dirigé vers la joue média de la trochlée.

L'axe longitudinal de l'élément tubulaire 68 de la représentation 3D de l'ancillaire correspondra sensiblement à l'évidement dirigé vers la surface pré-spinale de la face supérieure de l'extrémité proximale du tibia.

Ainsi, et tel que représenté à la Figure 10, des modifications locales apportées sur la représentation 3D de l'articulation tibio-fémorale, les formes et les dimensions de la surface d'ancrage de l'implant vont pouvoir être déterminées en fonction de la forme et des dimensions des modifications locales ainsi apportées, ici les évidements 612, 622 réalisés sur la représentation 3D de l'articulation tibio-fémorale, par soustraction du volume de l'implant 60 afin que la surface d'ancrage de l'implant permettent l'ancrage de l'implant sur la partie osseuse lésée.

Une fois la surface d'ancrage réalisée, la surface externe de l'implant va être déterminée.

La surface fonctionnelle de la représentation 3D de l'implant ancré sur l'extrémité distale fémorale 20 va être déterminée en fonction de la surface conjuguée de l'extrémité proximale tibiale 30 avec laquelle elle coopère et ce afin d'assurer l'articulation entre l'extrémité distale fémorale 20 et l'extrémité proximale tibiale 30, la surface conjuguée correspondant à la surface externe fonctionnelle de l'implant.

De la même façon, la surface fonctionnelle de la représentation 3D de l'implant ancré l'extrémité proximale tibiale 30 va être déterminée en fonction de la surface conjuguée de l'extrémité distale fémorale 20 avec laquelle elle coopère et ce afin d'assurer l'articulation entre l'extrémité distale fémorale 20 et l'extrémité proximale tibiale 30, la surface conjuguée correspondant à la surface externe fonctionnelle de l'implant.

L'articulation entre l'extrémité distale fémorale 20 et l'extrémité proximale tibiale 30 est avantageusement récupérée grâce à la prothèse de resurfacage du genou ainsi obtenu et a pour mission de remplacer à la fois les bandes usées de roulement des condyles qui roulent, glissent et tournent sur les surfaces glénoïdes, elles aussi usées, des plateaux tibiaux, sans toucher en rien à l'équilibre mécanique de l'articulation.

## Revendications

1. Procédé de fabrication d'un implant sur-mesure (11, 12) destiné à être implanté sur un site de pose d'au moins une partie osseuse lésée, **caractérisé en ce qu'**il comprend les étapes suivantes :
i. acquisition d'une ou plusieurs images d'au moins ladite partie osseuse lésée ;
ii. représentation graphique en 3D de l'imagerie d'au moins ladite partie osseuse lésée acquise à l'étape i. ;
iii. superposition d'une représentation 3D d'un implant standard sur la représentation 3D obtenue à l'étape ii., en positionnant ledit implant standard sur un site de pose à la surface de ladite au moins une partie osseuse lésée, modification des dimensions et/ou ajustement de la forme dudit implant standard en tenant compte d'au moins un paramètre de ladite au moins une partie osseuse lésée, puis modification d'une surface externe (112, 122) dudit implant standard;
iv. réalisation de l'implant sur mesure à partir des paramètres définitifs dudit implant obtenu à l'étape iii.;
**caractérisé en ce que** la modification de ladite surface externe dudit implant standard consiste à conférer audit implant standard ainsi positionné sur ledit site de pose, une surface fonctionnelle, lorsque ledit implant sur-mesure est destiné à être mis en œuvre à l'interface de deux parties osseuses coopérant l'une avec l'autre, ladite surface fonctionnelle étant déterminée de sorte qu'étant au moins partiellement en contact avec une surface conjuguée de l'autre partie osseuse avec laquelle ladite partie osseuse lésée coopère, elle assure l'articulation desdites parties osseuses, la détermination de ladite surface fonctionnelle est réalisée tandis que les deux parties osseuses sont en position fonctionnelle dans la représentation graphique 3D, une acquisition d'une ou plusieurs images desdites deux parties osseuses ayant été réalisée à l'étape i.

2. Procédé de fabrication selon la revendication 1, **caractérisé en ce que** ladite superposition d'une représentation 3D d'un implant standard sur la représentation 3D obtenue à l'étape ii. est réalisée par fondu.

3. Procédé de fabrication selon la revendication 1 ou 2, **caractérisé en ce qu'**avant l'étape de la détermination de la surface externe dudit implant, l'étape iii. comprend une étape dans laquelle au moins une modification locale directement est réalisé sur un site de pose de la représentation graphique en 3D de ladite au moins une partie osseuse lésée, une détermination de la forme et des dimensions de ladite surface d'ancrage d'un implant en fonction de la forme et des dimensions de ladite au moins une modification locale est réalisée sur ladite représentation graphique 3D, ladite surface d'ancrage permettant l'ancrage de celui-ci sur ladite partie osseuse lésée puis détermination de la surface externe dudit implant.

4. Procédé de fabrication selon l'une quelconque des revendications 1 à 3, **caractérisé en ce que** ledit implant est une partie d'un dispositif médical destiné à être implanté.

5. Procédé de fabrication selon l'une quelconque des revendications 1 à 4, **caractérisé en ce que** ladite surface conjuguée correspond à la surface externe d'un autre implant reçu à la surface de ladite autre partie osseuse coopérant avec ladite partie osseuse lésée.

6. Procédé de fabrication selon l'une quelconque des revendications 1 à 5, **caractérisé en ce que** la surface externe dudit implant est déterminée par enlèvement de matière d'une surface pleine de la représentation graphique de l'implant.

7. Procédé de fabrication selon la revendication 6, **caractérisé en ce qu'**à l'étape iii. a), la détermination de la portion de l'implant à enlever est déterminée par soustraction de la seule représentation graphique de ladite au moins une partie osseuse à réparer obtenue à l'étape ii. à la représentation graphique représentant l'ensemble partie osseuse et implant standard positionné sur son site de pose.

8. Procédé de fabrication selon l'une quelconque des revendications 1 à 7, **caractérisé en ce que** la surface externe dudit implant est déterminée par ajout de matière.

9. Procédé de fabrication selon l'une quelconque des revendications 1 à 8, **caractérisé en ce qu'**à l'étape iii. b) ladite modification locale est un évidemment continu.

10. Procédé de fabrication selon l'une quelconque des revendications 1 à 9, **caractérisé en ce qu'** à l'étape iv), ledit implant est réalisé par fabrication additive.

11. Procédé de fabrication selon l'une quelconque des revendications 1 à 10, **caractérisé en ce qu'**il comprend une étape supplémentaire de fabrication additive de ladite au moins une partie osseuse acquise à l'étape ii.

12. Programme d'ordinateur comportant des instructions pour l'exécution du procédé de fabrication d'un implant sur mesure selon l'une quelconque des revendications 1 à 11, lorsque le programme est exécuté par un processeur.

13. Support d'enregistrement sur lequel est stocké le programme d'ordinateur selon la revendication 12.

## Patentansprüche

1. Verfahren zur Herstellung eines maßgeschneiderten Implantats (11, 12), das dazu bestimmt ist, an einer Implantationsstelle wenigstens eines beschädigten Knochenabschnitts implantiert zu werden, **dadurch gekennzeichnet, dass** es die folgenden Schritte umfasst:
i. Erfassung eines oder mehrerer Bilder wenigstens des beschädigten Knochenabschnitts;
ii. grafische Darstellung in 3D der in Schritt i. aufgenommenen Bilder wenigstens des beschädigten Knochenabschnitts;
iii. Überlagerung einer 3D-Darstellung eines Standardimplantats mit der in Schritt ii. erhaltene 3D-Darstellung, wobei das Standardimplantat an einer Implantationsstelle auf der Oberfläche des wenigstens einen beschädigten Knochenabschnitts positioniert wird, wobei die Abmessungen des Standardimplantats unter Berücksichtigung wenigstens eines Parameters des wenigstens einen beschädigten Knochenabschnitts geändert und/oder die Form des Standardimplantats angepasst wird und anschließend eine Außenfläche (112, 122) des Standardimplantats geändert wird;
iv. Herstellung des maßgeschneiderten Implantats anhand der endgültigen Parameter des in Schritt iii. erhaltenen Implantats;
**dadurch gekennzeichnet, dass** die Änderung der Außenfläche des Standardimplantats darin besteht, dem so an der Implantationsstelle positionierten Standardimplantat eine Oberfläche zu verleihen, die funktionsfähig ist, wenn das maßgefertigte Implantat dazu bestimmt ist, an der Schnittstelle zweier miteinander zusammenwirkender Knochenteile eingesetzt zu werden, wobei die funktionsfähige Oberfläche so bestimmt ist, dass sie, wenn sie wenigstens teilweise mit einer Gegenfläche des anderen Knochenabschnitts, mit dem der beschädigte Knochenabschnitt zusammenwirkt, in Kontakt steht, die gelenkige Verbindung der Knochenabschnitte gewährleistet, wobei die Bestimmung der Funktionsfläche erfolgt, während sich die beiden Knochenabschnitte in der 3D-Darstellung in ihrer Funktionsposition befinden, wobei in Schritt i eine Aufnahme eines oder mehrerer Bilder der beiden Knochenabschnitte erfolgt ist.

2. Herstellungsverfahren nach Anspruch 1, **dadurch gekennzeichnet, dass** das Überlagern einer 3D-Darstellung eines Standardimplantats mit der in Schritt ii. erhaltenen 3D-Darstellung durch Überblenden erfolgt.

3. Herstellungsverfahren nach Anspruch 1 oder 2, **dadurch gekennzeichnet, dass** vor dem Schritt der Bestimmung der Außenfläche des Implantats der Schritt iii. einen Schritt umfasst, in dem wenigstens eine lokale Modifikation direkt an einer Implantationsstelle der grafischen 3D-Darstellung des wenigstens einen beschädigten Knochenabschnitts vorgenommen wird, eine Bestimmung der Form und der Abmessungen der Verankerungsfläche eines Implantats in Abhängigkeit von der Form und den Abmessungen der wenigstens einen lokalen Modifikation an der dreidimensionalen grafischen Darstellung durchgeführt wird, wobei die Verankerungsfläche die Verankerung desselben an dem beschädigten Knochenabschnitt ermöglicht, und anschließend die Außenfläche des Implantats bestimmt wird.

4. Herstellungsverfahren nach einem der Ansprüche 1 bis 3, **dadurch gekennzeichnet, dass** das Implantat Teil einer medizinischen Vorrichtung ist, die zur Implantation bestimmt ist.

5. Herstellungsverfahren nach einem der Ansprüche 1 bis 4, **dadurch gekennzeichnet, dass** die konjugierte Fläche der Außenfläche eines anderen Implantats entspricht, das an der Oberfläche des anderen Knochenabschnitts aufgenommen ist, der mit dem beschädigten Knochenabschnitt zusammenwirkt.

6. Herstellungsverfahren nach einem der Ansprüche 1 bis 5, **dadurch gekennzeichnet, dass** die Außenfläche des Implantats durch Entfernen von Material von einer vollen Fläche der grafischen Darstellung des Implantats bestimmt wird.

7. Herstellungsverfahren nach Anspruch 6, **dadurch gekennzeichnet, dass** in Schritt iii. a) die Bestimmung des zu entfernenden Teils des Implantats durch Subtraktion der einzigen grafischen Darstellung des wenigstens einen zu reparierenden Knochenabschnitts, die in Schritt ii. erhalten wurde, von der grafischen Darstellung, die den Knochenabschnitt und das an seiner Implantationsstelle positionierte Standardimplantat darstellt, erfolgt.

8. Herstellungsverfahren nach einem der Ansprüche 1 bis 7, **dadurch gekennzeichnet, dass** die Außenfläche des Implantats durch Hinzufügen von Material bestimmt wird.

9. Herstellungsverfahren nach einem der Ansprüche 1 bis 8, **dadurch gekennzeichnet, dass** in Schritt iii. b) die lokale Modifikation eine durchgehende Aussparung ist.

10. Herstellungsverfahren nach einem der Ansprüche 1 bis 9, **dadurch gekennzeichnet, dass** in Schritt iv) das Implantat durch additive Fertigung hergestellt wird.

11. Herstellungsverfahren nach einem der Ansprüche 1 bis 10, **dadurch gekennzeichnet, dass** es einen zusätzlichen Schritt der additiven Herstellung des in Schritt ii gewonnenen wenigstens einen Knochenabschnitts umfasst.

12. Computerprogramm, umfassend Anweisungen zur Durchführung des Verfahrens zur Herstellung eines maßgeschneiderten Implantats nach einem der Ansprüche 1 bis 11, wenn das Programm von einem Prozessor ausgeführt wird.

13. Speichermedium, auf dem das Computerprogramm nach Anspruch 12 gespeichert ist.

## Claims

1. Method for manufacturing a custom-made implant (11, 12) intended to be implanted in a site of placement of at least one injured bone part, **characterised in that** it comprises the following steps:
i. acquiring one or more images of at least said injured bone part;
ii. a 3D graphical representation of the imaging of at least said injured bone part acquired in step i.;
iii. superimposing a 3D representation of a standard implant on the 3D representation obtained in step ii., positioning said standard implant on a placement site on the surface of said at least one injured bone part, modifying the dimensions and/or adjusting the shape of said standard implant taking into account at least one parameter of said at least one injured bone part, then modifying an external surface (112, 122) of said standard implant;
iv. making the custom-made implant from the final parameters of said implant obtained in step iii.;
**characterised in that** the modification of said outer surface of said standard implant consists in conferring on said standard implant thus positioned on said placement site, a functional surface, when said custom-made implant is intended to be used at the interface of two bone parts cooperating with one another, said functional surface being determined such that, being at least partially in contact with a conjugate surface of the other bone part with which said injured bone part cooperates, it ensures the articulation of said bone parts, the determination of said functional surface is carried out while the two bone parts are in a functional position in the 3D graphical representation, an acquisition one or more images of said two bone parts having been made in step i.

2. Manufacturing method according to claim 1, **characterised in that** said superposition of a 3D representation of a standard implant on the 3D representation obtained in step ii. is carried out by fading.

3. Manufacturing method according to claim 1 or 2, **characterised in that**, before the step of determining the outer surface of said implant, step iii. comprises a step in which at least one local modification is performed directly on a site of placement of the 3D graphical representation of said at least one injured bone part, a determination of the shape and dimensions of said anchoring surface of an implant according to the shape and dimensions of said at least one local modification is performed on said 3D graphical representation, said anchoring surface allowing anchoring thereof on said injured bone part then determining the external surface of said implant.

4. Manufacturing method according to any one of claims 1 to 3, **characterised in that** said implant is a part of a medical device intended to be implanted.

5. Manufacturing method according to any one of claims 1 to 4, **characterised in that** said conjugate surface corresponds to the outer surface of another implant received on the surface of said other bone part cooperating with said injured bone part.

6. Manufacturing method according to any one of claims 1 to 5, **characterised in that** the outer surface of said implant is determined by removing material from a solid surface of the graphical representation of the implant.

7. Manufacturing method according to claim 6, **characterised in that** in step iii. a), the determination of the part of the implant to be removed is determined by subtracting the only graphical representation of said at least one bone part to be repaired obtained in step ii. from the graphical representation representing the bone part and standard implant assembly positioned at its placement site.

8. Manufacturing method according to any one of claims 1 to 7, **characterised in that** the outer surface of said implant is determined by adding material.

9. Manufacturing method according to any one of claims 1 to 8, **characterised in that** in step iii. b) said local modification is a continuous recess.

10. Manufacturing method according to any one of claims 1 to 9, **characterised in that** in step iv), said implant is produced by additive manufacturing.

11. Manufacturing method according to any one of claims 1 to 10, **characterised in that** it comprises an additional step of additive manufacturing of said at least one bone part acquired in step ii.

12. Computer program comprising program code instructions for executing the method according to any one of claims 1 to 11 when said program is executed on a computer.

13. Recording medium on which the computer program according to claim 12 is stored.
